# EUROPEAN PATENT SPECIFICATION

(11) **EP 2 200 765 B1**
(45) Date of publication and mention of the grant of the patent: **27.03.2019**
(21) Application number: 08741776.2
(22) Date of filing: 24.04.2008
(51) Int. Cl.: B21D 11/00, A61F 2/30, G06T 7/00, G06T 17/00

(54) **SYSTEM FOR AUTOMATIC AND PERSONALIZED MODELLING/BENDING OF SURGICAL PROSTHESIS FOR CORRECTION OF PECTUS EXCAVATUM BASED ON PRE-SURGICAL IMAGING INFORMATION**
SYSTEM ZUM AUTOMATISCHEN UND PERSONALISIERTEN MODELLIEREN/BIEGEN EINER CHIRURGISCHEN PROTHESE ZUR KORREKTUR EINES PECTUS EXCAVATUM AUF GRUNDLAGE VON PRÄCHIRURGISCHEN ABBILDUNGSINFORMATIONEN
SYSTÈME DE MODELAGE/FLEXION AUTOMATIQUE ET PERSONNALISÉ D'UNE PROTHÈSE CHIRURGICALE DESTINÉE À LA CORRECTION DU THORAX EN ENTONNOIR SUR LA BASE D'INFORMATIONS D'IMAGERIE PRÉCHIRURGICALES

(30) Priority: 13.09.2007 PT 103823
(43) Date of publication of application: 30.06.2010
(73) Proprietor: Universidade do Minho, 4704-320 Braga (PT)
(72) Inventor: MARTINS VILAÇA, João Luís Araújo, P-4705-671 Tadim - Braga (PT); MARQUES PINHO, António Costa, P-4800-056 Guimarães (PT); CORREIA PINTO, Jorge, P-4100-053 Porto (PT); CRUZ FONSECA, Jaime Francisco, P-4700-752 Palmeira (PT); MAIA PEIXINHO, Nuno Ricardo, P-4810-231 Guimarães (PT)
(74) Representative: Ferreira, Maria Silvina
(86) International application number: PCT/PT2008/000016
(87) International publication number: WO 2009/035358

(56) References cited:
- US-A- 6 024 759
- US-A1- 2004 243 481
- US-A1- 2005 262 911
- FERRETTI G R ET AL: "Virtual tools for imaging of the thorax." THE EUROPEAN RESPIRATORY JOURNAL : OFFICIAL JOURNAL OF THE EUROPEAN SOCIETY FOR CLINICAL RESPIRATORY PHYSIOLOGY AUG 2001, vol. 18, no. 2, August 2001 (2001-08), pages 381-392, XP002495743 ISSN: 0903-1936
- PRETORIUS E S ET AL: "Spiral CT with 3D reconstruction in children requiring reoperation for failure of chest wall growth after pectus excavatum surgery. Preliminary observations." CLINICAL IMAGING 1998 MAR-APR, vol. 22, no. 2, March 1998 (1998-03), pages 108-116, XP002495744 ISSN: 0899-7071
- RUSHING ET AL: "When it is not an infection: metal allergy after the Nuss procedure for repair of pectus excavatum" JOURNAL OF PEDIATRIC SURGERY, W. B. SAUNDERS COMPANY, US, vol. 42, no. 1, 17 January 2007 (2007-01-17), pages 93-97, XP005821080 ISSN: 0022-3468

## Description

### Scope of the invention

The present invention was conceived to optimize the personalized modelling and bending of thoracic prosthesis to apply in the correction of *Pectus Excavatum.*

### State of the art

*Pectus excavatum* is a deformity of the thoracic wall that, in a high percentage of cases, demands surgical correction. The classic surgical technique, assigned as Ravitch technique was, in the last decade, gradually substituted for surgical techniques that involve the placement of a convex thoracic prosthesis in sub-sternal position, initially described by Donald Nuss (US6024759). Briefly, the surgical technique consists of placing thoracic prosthesis in sub-sternal position through bilateral cutaneous incisions in the lateral thoracic wall (Nuss et al, J Pediatr Surg 1998). This technique has shown to be less aggressive, faster to execute and allows better cosmetic results. However, this technique estimates the thoracic application of one prosthesis that has to be modelled/bent in accordance with the thoracic morphology of each patient. Such is fulfilled per operatively and manually following the form of a mould generated during the surgical procedure. This procedure is slow, fastidious for the surgical team and leaves imperfections, many times, in the surface of the prosthesis. The main imperfections are the non uniform strength distribution of the prosthesis in the support area (ribs), which concern initial discomfort thus increasing the adaptation time of the patient to the prosthesis. On the other hand, one third of the surgical time of the Nuss technique is dedicated to the modelling and bending of this prosthesis.

The present invention minimizes the previously cited inconveniences. In the pre-operative study of this type of patients, it is routine, to perform some other complementary examinations to reach the diagnosis, such as a CT-scan of the thoracic wall. The equipment herein described allows, on the basis of the information of the thoracic CT-Scan, to select the size, and automatically model/fold pre-operatively the thoracic prosthesis to apply. Such, allows the application of a personalized prosthesis which makes possible that the application of the forces in the zone of support of surgical prosthesis to be uniformly distributed, attenuating in such a way, the discomfort initially provoked and therefore shortening the period of adaptation to the same one. One should also enhance the significant shortening of the duration of the surgery, and the possibility of the patient having a virtual perspective of the post-operative result foreseen before the surgical intervention.

The prior art document US2005262911 discloses a computer-aided system for on the fly design and automated manufacture of implants. It concerns a combination of manufacturing hardware and computer-aided design system used to imposed desired structure on an article for surgical use. However, the prior art document does not disclose a process to create a personalized virtual mould of the surgical prosthesis to apply in the patient based on anatomo-surgical references that allows the simulation of the pre-operative, expected cosmetic result of the thoracic wall after placing the surgical prosthesis in the patient, and bending the surgical prosthesis.

The use of systems of medical imaging for the attainment of models of reference of prosthesis is already described for particular cases, for example: production of active biological prosthesis for the reconstruction of bone tissue (CA2561727); drawing and production to the measure of surgical tool-guide for the implantation of bone prosthesis (US2005148843); attainment of prosthesis geometry of spinal disc (WO0180786). The system herein presented stands out by the particular application in modelling and bending of prosthesis for correction of *pectus excavatum* and by establishing a complete and integrated system of modelling, attainment of personalized geometry, and pre-operative modelling and bending appealing to specific equipment and software belonging to the owner.

### Summary of the Invention

The present invention relates to a system of modelling and bending of surgical prothesis used in the correction of *pectus excavatum*, allowing its pre-operative personalization according to the thoracic morphology of each patient. The system allows, on the basis of the information of the thoracic CT-scan, the application of a personalized prosthesis, reducing the initial discomfort provoked by the prosthesis and subsequent period of adaptation to the same, allowing still to reduce the duration of the surgery, and offering the patient a virtual perspective of the foreseen postoperative result.

For the effect, the system is constituted by:
- Simulation and modelling of surgical prosthesis, supported in software modules, developed specifically for the effect, being executed in a personal computer;
- Command and control of the machine, supported in software modules that are executed in a controller;
- An equipment that includes electronic and mechanical components, for the bending of personalized surgical prosthesis, to apply in the patient.

The software modules that are executed in the personal computer have a friendly interface with the user, mainly a set of menus that guide it step by step to reach the intended goal - modelling the surgical prosthesis and simulation of the cosmetic result of its application in the patient. After the process of modelling and simulation, this application generates a set of points of control that will be sent to the controller. This includes software modules that allow receiving the control points sent by the application from the personal computer and based on this information commands and controls the equipment in an adaptive manner in the process of bending of surgical prosthesis.

### Brief description of the drawings

Many of the aspects of the invention can be better understood regarding some drawings on the same. The components in the drawings are not necessarily to scale, emphasis is placed instead in the clear illustration of the principles of the present invention. The drawings are included with a non-limitative character, merely with the objective to provide a better understanding of the following description:
Figure 1: Represents a general three-dimensional perspective of an embodiment of the machine for the bending according to the invention.
Figure 2: Represents the general scheme of the different modules of the modelling and bending system.
Figure 3: Corresponds to a detailed bidimensional representation of the main body of the modelling and bending equipment.
Figure 4: Represents a detailed three-dimensional perspective of the head of the modelling equipment.
Figure 5: Represents a three-dimensional perspective of the equipment for modelling and bending of the surgical prosthesis.
Figure 6: Represents the general block diagram of the algorithm of the software application developed for the personal computer.

### Detailed description of the invention

The present invention will now be described in detail, as a non-limitative example, by means of a preferred embodiment, represented in the enclosed drawings.

In reference to the figures, the preferred embodiment of the invention is described where the equipment is constituted by the following set of basic elements is described in detail: engines (1 and 2); main body of the machine (3); head of the machine (4); controller (9) and personal computer (8).

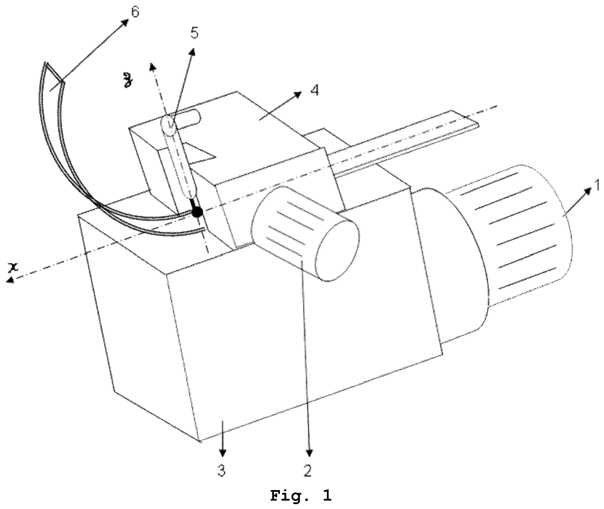

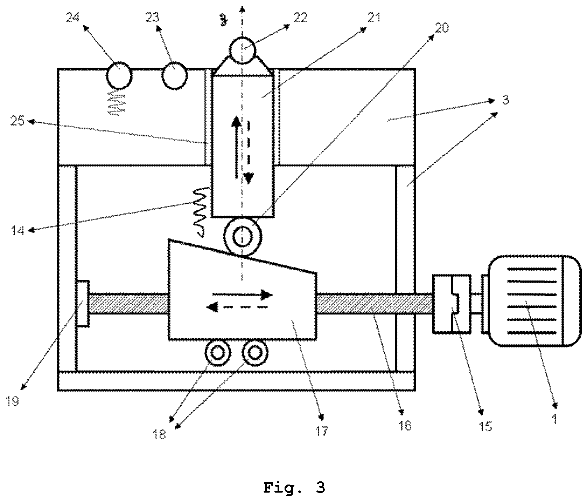

Electromechanical commands (see figures 1, 2 and 3) - the equipment has two engines (1 and 2). One of them (1) is connected to the main body of the machine (3) and undertakes the task of bending surgical prosthesis (6). The motor (2) is connected to the head of the machine (4) and its function is to execute the advance of the referred prosthesis.

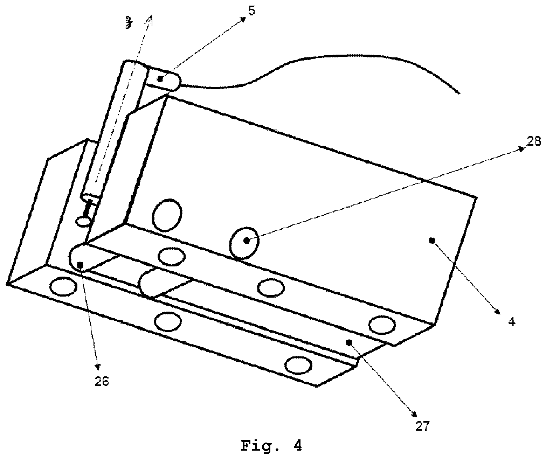

Machine's main body (see figures 1, 3 and 4) - This is the part of the equipment where the great effort of bending is developed, necessary to the bending of surgical prosthesis(6). For the effect, the pivoting movement of the engine (1), is transformed into a translation movement, in the radial direction - axle z - to automatically adapt itself to the bending process requirements. That is obtained due to a system of ramp/piston (17 and 21).

The ramp (17), by a thread system (16), connected to the engine (1) through a universal joint (15), depending on the pivoting direction and speed received, assumes a two-way translation movement and actuates the piston (21), in the radial direction, axle z.

All the components of this mechanism, submitted to movements, are appropriately guided by bearing systems of rolling or slipping (18,19,20 and 25) and can be considered movements without friction or, if present, they can be ignored, in the scope of the efforts involved in the system. So being, high efforts, which are required in bending process of surgical prosthesis (6), are obtained.

It is still considered permissible the use of conventional mechanisms for this binomial of pivoting/translation movement such as: connecting rod/crankshaft; latch of moulds; screw/nuts and linear table; among others. There is further a system of springs (14) that guarantees the permanent contact between the piston (21) and the ramp (17).

For an efficient electronic control of the equipment, the existing movements in the machine's body (3) are in permanent contact with the controller (9), through electric sensors (5, 7, 12 and 13) here integrated. For the translation or drag movement of the surgical prosthesis (6) - axle x -, the main body of the equipment (3) holds two of the three shafts required to this movement (23 and 24), strategically placed such as to allow that surgical prosthesis (6) moves without the interfering with the static parts.

In this manner, the friction effect between the static parts of the body (3) and the head of the machine (4) with the surgical prosthesis (6) is prevented, when it is submitted to the translation movement. One of them (23) is connected to the advance motor (2) that for friction imposes the drag of the surgical prosthesis (6) - pivoting versus translation movement. The other shaft (24) is damped, which allows that when the piston (21) is retreated, the surgical prosthesis (6) is exclusively supported in the three lodes and is easily dragged by the motor (2).

On the other hand, the damping system of this shaft (24), allows a better static support of the surgical prosthesis (6) when this is submitted to the bending effort. The third shaft (28) is integrated in the head of the machine (4) and only serves as necessary support to the movement of translation of the surgical prosthesis (6).

The material of the surgical prosthesis(6) obeys the mechanical principles of the material, that identifies two deformation behaviours namely: tensile deformation and plastic or permanent deformation. The first one - tensile - refers to the deformation that is recovered and the second one - plastic - refers to the irrecoverable deformation, which means, to the deformation (or bending) definitively assumed, in a certain point, for the surgical prosthesis (6) after the retreat of the piston (21).

Herein resides another main objective of the present patent, that is if the material behaviour in mechanical processing is not previously recognized, nor would it be possible - because, according to the state of the art, the material behaviour is intrinsically associated with a set of metallurgical and physical/mechanical parameters which are responsible for the fact that, in a same lot of the same hypothetical material, the mechanical behaviour is different, being therefore necessary that the system recognizes in each instant and in an adaptable way, the permanent deformation to which the surgical prosthesis (6) was submitted.

The materials used in the manufacture of this part of the equipment are compatible with the material to process - surgical prosthesis (6). To the main body of the machine (3) the motors (1 and 2) and the head of the machine (4) are also attached

Head of the machine (see figures 1,2 and 4) - the assignment given to this part of the equipment is enlightening as far as its functionality is concerned, that is, it is in this part that the bending of surgical prosthesis (6) is mechanically processed. Herein, the surgical prosthesis (6) is introduced in a channel (27), specifically opened for the effect, which by means of the motor movement (2), displaces the surgical prosthesis (6) in direction X (see figure 1), towards the position previously defined by the controller (9).

After the automatic positioning of the surgical prosthesis (6), the controller (9) makes the piston advance (21), through the motor (1), in the direction perpendicular to the surgical prosthesis axle (6) - axle z - which, in this way, bends the surgical prosthesis (6). For this effect a pin (26) attached to the head of the machine is provided, that restricts the surgical prosthesis movement (6) in the direction of the axle z.

Likewise, and in the piston end (21), there is a pin (22), manufactured in a material that is compatible with the one surgical prosthesis (6) meterial, which facilitates its bending. The precision required for the bending is high. Such was obtained through the real time control of the behaviour of the material, which means, a displacement sensor (5) was integrated in the head of the machine (4) controlling in real time through a software application, specifically developed for the effect, the mechanical behaviour of the material referred to in the previous point. Therefore, it is possible to identify the tensile deformation parcels (recoverable) and permanent deformation parcels (of bending).

Such software application has is based on an algorithm that is divided into two parts. The first part of the algorithm is executed in the first bending point of the surgical prosthesis (6), and is characterized by the determination of the value corresponding to the plastic deformation of the material. This value is obtained through cycles of successive advances (incremental) and retreats (total) of the piston (21), towards to the surgical prosthesis (6). The stop condition of the cycle is given when displacement sensor (5) detects permanent deformation in the surgical prosthesis (6) at the moment that the piston is retreated. The second part of the algorithm is applied in each bending point of the surgical prosthesis (6), and is composed by two iterations. Each iteration consists of an advance movement and a retreat movement of the piston (21). For the first iteration the advance value of the piston (21) is given by the addition of each bending point value to the plastic deformation value, previously calculated, to less than one constant (return factor - experimentally obtained). The use of a plastic deformation value inferior to the one calculated is related with the fact that the material obeys to a tension/deformation law that is not linear. For the second iteration, the advance value of the piston (21) is obtained in a weighed manner between the advance values of the first iteration and the required and effective bending values, after the retreat movement of the piston (21) in the first iteration.
The materials used in the production of this part of the equipment are compatible with the material to process.

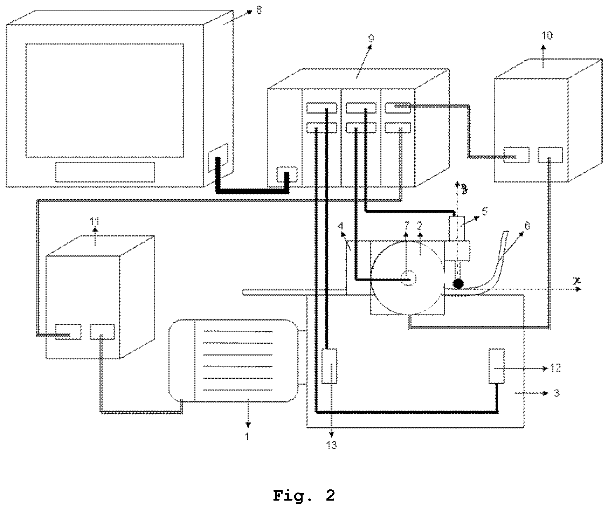

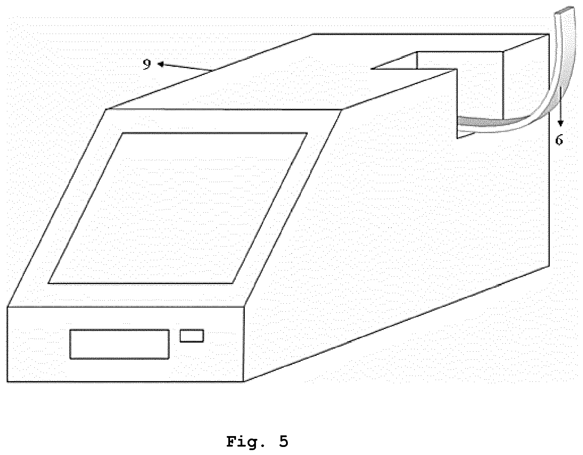

Controller (see figure 2 and 5) - Figure 2 presents a general scheme of the different constituent blocks of the equipment, where a personal computer (8) emerges for an interface with the user (8). This allows the user to introduce the data that refers to the information of the thoracic CT-Scan (medical images) of the patient, and to get, through the software, a 3D representation of the profile and dimension of the surgical prosthesis (6), the localization and orientation to place the prosthesis in the intercostal space and perform a simulation of the cosmetic effect of the application of the prosthesis.

From the information obtained from the profile and dimension of the prosthesis virtually shaped, the developed software sends through the proprietor protocol a set of control points for the cited controller (9). This controller is responsible for the following set of functionalities: interpretation and translation of the control points sent by the personal computer (8); synchronization and control of the axles xx and zz of the equipment; validation of the physical safety conditions of the same. For communication between the personal computer (8) and the controller (9) a communication protocol was implemented that has the protocol TCP/IP (Transfer Protocol Control/Internet Protocol) as its base, where a socket connection between the personal computer (8) and the controller (9) is established. In the last ones, an application layer was developed that defines a set of services such as the message format, the error correction code and the confirmation of a well-succeeded reception of the messages. These messages are of two types: status and data or information upload. The first gives information concerning the status of the two interlocutors (busy or free). The second sends information concerning the control points from the personal computer (8) to the controller (9). Thus, for example, when the user asks the computer's (8) resident software to actuate the bending of the surgical prosthesis (6), a message is sent to the controller (9) informing that the bending process is about to start. The controller (9) might answer in two ways: that it is free to start the process or that it is busy. In the first case, the personal computer's resident software (8) starts the data transmission to the controller (9) (control points); in the second case, the software sends a message to the user, informing that the controller (9) is busy and asks him to retry later. The use of this protocol allows the remote operation of the equipment, allowing its use via Web.

The equipment axles - movement/deformation directions of the surgical prosthesis (6) - are controlled in two different ways. The xx axle is constituted by the motor (2) that provides it with movement and whose lode is connected to an "encoder" (7). The later is connected to the fast inlets of the controller (9). The motor (2) is actuated by a power driver (10) that is commanded by the controller (9), through a letter of digital oulets, thus closing in the control cycle. The movement through the zz axle is established by a motor (1) that provides it with movement and a displacement sensor (5). The later is connected to the controller (9) through analogical border entrances allowing the reading of the effective deformation of the surgical prosthesis (6) in the bending process. The motor (1) is set in motion through a power driver (11), which is commanded through the controller (9), by means of a letter of digital outlets. The control cycle herein implemented confers to the system a quality control of the bent prosthesis.

To assure the safety of the elements of the machine, the zz axle is protected by two movement sensors (12 and 13), that function as limits of end of course, the sensor (13) also functioning as an initial position sensor. These sensors (12, 13) are connected to the controller (9), by means of a letter of digital inlets.

The detection of the prosthesis by the equipment is given by the reading of the sensor (5), and the adjustment of the same for the initial position is made automatically, using for such a control mesh made by the motor (2), the controller (9) and closed by the sensor (5).

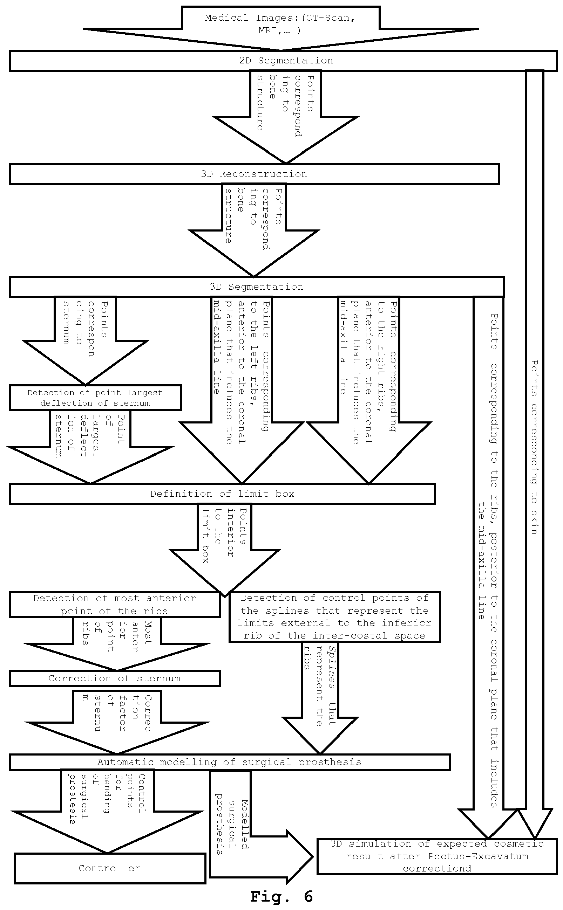

Personal computer (see figure 2 and 6) - the interface between the equipment and the user is made through a software application that runs in a personal computer (8). This has a pleasant interface comprising a set of menus that guide the user step by step to reach the intended objective.

In this software application several 2D and 3D image processing techniques were used, of which the following two are outlined: 2D segmentation, that allows separating different structures based on colour differences at the points that are part of thoracic CT-Scan images; 3D segmentation, that allows separating different structures based on the spatial position of the points that are part of the image and in the identification of anatomo-surgical references - coronal plane and mid-axilla line. Within this scope, the software application allows the graphical 3D pre-operative simulation of the expected cosmetic result of the thoracic wall after placement of the surgical prosthesis (6) in the patient. In the pre-operative stage the points that represent the skin structure are used to reconstruct the three-dimensional model of the thoracic wall. This model is represented as surfaces with normal orientation. Features such as the bar placement point (obtained from the point of largest deflection of the sternum), correction factor of sternum (obtained from the most anterior point of ribs in the limit area) and stabilization factor of the sternum (corresponding to the displacement of the sternum after removal of the bar) are used to recalculate the position of each point associated to the surfaces that represent the skin affected by the correction of the malformation. The skin points directly associated to the spatial position of the sternum are directly affected by the correction factor of the sternum. The neighbour points, herein referred as neighbour pectus, are affected by a matricial Gaussian distribution.
This application software has the following set of features:
- Reading of neutral files universally used in medical imaging such as those resulting from CT-Scan and magnetic resonance systems;
- Identification, based on 2D image processing techniques, of the relevant poins corresponding to the bone structure and the ones representing skin structure;
- Conversion of 2D images in 3D images based on conventional image processing techniques;
- Identification, based on 3D image processing techniques implemented through proprietary algorithms, of the following four structures: points corresponding to the ribs, posterior to the coronal plane that includes the mi-axilla line; points corresponding to the right ribs anterior to the coronal plane that includes the mid-axilla line; points corresponding to the left ribs anterior to the coronal plane that includes the mid-axilla line; points corresponding to the sternum;
- automatic detection of the point of largest deflection of the sternum;
- definition of the intercostal space; sternum points; right and left ribs interior to the limit box, with the lower base sitting on the plane that corresponds to the point of largest deflection of the sternum, and with the top base sitting on a plane parallel to the anterior plane and at a distance equal to the width of the surgical prosthesis (6);
- automatic correction of the sternum elevation to a predicted position, given by the correction factor of the sternum;
- possibility for the user to easily change, manually, the predicted position of the sternum;
- selection of the most adequate surgical prosthesis (6) to apply in the patient from standard sizes, based on the position defined for the sternum;
- positioning and visualizing the virtual surgical prosthesis modelled from the information previously determined: point of largest deflection and final position of the sternum,
- 3D visualization/simulation of the possible result to obtain with the placement of the surgical prosthesis (6);
- Automatic generation/selection of the control points to send to the controller (9) for subsequent bending of the surgical prosthesis (6);
- Sending of control points to the controller (9)using proprietary protocol.

The controller (9) and personal computer's (8) software applications were developed specifically for this modelling and bending system of surgical prosthesis used in the correction of *pectus excavatum.*

It must be clear that the automatic modelling and bending system of personalized surgical prosthesis (6) for correction of *pectus excavatum* based on pre-surgical imaging information previously described is merely a possible implementation example established for a clear understanding of the principles of the invention. Variations and modifications can be made to the embodiment previously described. All these modifications and variations must be included in the subject of the scope of the present invention and be protected by the following claims.

## Claims

1. Process for pre-surgical, automatic and personalized modelling and bending of a surgical prosthesis (6) for use in the correction of *pectus excavatum* **comprising** the following steps:
a) segmenting from 3D medical images of a patient, the structures comprising the ribs and the sternum based on anatomo-surgical references;
b) automatically detecting the point of largest deflection of the sternum based on 3D coordinates of the points from the sternum;
c) defining the limit box where the intercostal space is located by:
defining a plane that includes the point of largest deflection of the sternum and is perpendicular to the sternum; creating a cuboid with one of the bases abutting on the defined plane and having height equal to the width of the prosthesis (6); defining the intercostal space through the points of the ribs interior to the cuboid;
d) automatic correction of the sternum elevation to a position predicted for the correction of *pectus excavatum*, given by the correction factor of the sternum obtained from the most anterior point of the ribs belonging to the intercostal space, having this position the possibility of later manual modification by the user;
e) virtual and personalized modelling of the surgical prosthesis (6) and selection of the most appropriate standard size of the prosthesis to be applied in the patient, based on the position defined for the sternum;
f) positioning and visualizing the virtual surgical prosthesis modelled from the point of largest deflection and final position of the sternum;
g) graphical simulation of the pre-operative expected cosmetic result of the thoracic wall after placement of the surgical prosthesis (6) in the patient;
h) bending of the surgical prosthesis (6), according to the virtual model, with real-time monitoring and control of the mechanical behavior of the surgical prosthesis (6) material.

2. Process, according to the previous claim, **wherein** the segmentation from the 3D images of the rib structure and sternum structure comprises the following steps:
a) identifying the coronal plane, that includes the mid-axillary line, that limits the anterior position of the ribs and consequently the initial and final point of the prosthesis (6);
b) identifying the medial plane that, based on limits determined experimentally on the left and on the right of the respective plane, allows the separation of the sternum from the ribs.

3. Process, according to any one of the previous claims, **wherein** the graphical pre-surgical simulation of the expected cosmetic result of the thoracic wall, after placement of the surgical prosthesis (6) in the patient, is obtained from information on the point of bar placement obtained from:
i) the point of largest deflection of the sternum;
ii) the correction factor of the sternum given by the most anterior point of the ribs present in the limit box;
iii) and by the stabilizing factor of the sternum that corresponds to the displacement of the sternum after removing the bar.

## Patentansprüche

1. Verfahren zur präoperativen, automatischen und personalisierten Modellierung und Biegung einer chirurgischen Prothese (6) zur Verwendung bei der Korrektur der Trichterbrust, das aus folgenden Schritten besteht:
a) Segmentierung der aus den Rippen und dem Brustbein bestehenden Strukturen aus dreidimensionalen medizinischen Bildern eines Patienten auf Basis von anatomischchirurgischen Referenzen;
b) automatische Erkennung des Punktes der größten Durchbiegung des Brustbeins auf Basis der 3D-Koordinaten der Punkte des Brustbeins;
c) Definition des Grenzbereichs, wo sich der Interkostalraum befindet, durch:
Definition einer Ebene, welche den Punkt der größten Durchbiegung des Brustbeins beinhaltet und senkrecht zum Brustbein steht; Erstellen eines Quaders mit einer der Basen, der an der definierten Ebene angrenzt und eine Höhe aufweist, die der Breite der Prothese (6) entspricht; Definition des Interkostalraums durch die Punkte der Rippen innerhalb des Quaders;
d) automatische Berichtigung der Brustbeinhöhe auf eine Position, die für die Korrektur der Trichterbrust vorgesehen ist, unter Berücksichtigung des Korrekturfaktors des Brustbeins, der vom vordersten Punkt der zum Interkostalraum gehörenden Rippen erhalten wurde, wobei diese Position die Möglichkeit einer späteren manuellen Änderung durch den Benutzer bietet;
e) virtuelle und personalisierten Modellierung der chirurgischen Prothese (6) und Auswahl der am besten geeigneten Standardgröße für die am Patienten angewandten Prothese auf Basis der für das Brustbein definierten Position;
f) Positionierung und Visualisierung der virtuellen chirurgischen Prothese, die auf Basis des Punktes der größten Durchbiegung und der endgültigen Position des Brustbeins modelliert wurde.
g) grafische Simulation des erwarteten präoperativen kosmetischen Resultats der Thoraxwand nach dem Einsetzen der chirurgischen Prothese (6) am Patienten;
h) Biegen der chirurgischen Prothese(6), gemäß dem virtuellen Modell mit Echtzeit-Überwachung und Kontrolle des mechanischen Verhaltens des Materials der chirurgischen Prothese (6).

2. Verfahren gemäß dem vorherigen Anspruch, worin die Segmentierung der 3D Bilder der Rippenstruktur und der Struktur des Brustbeins aus folgenden Schritten besteht:
a) Bestimmung der Frontalebene, welche die mittlere Axillarlinie beinhaltet, die die vordere Position der Rippen und folglich den Anfangs- und Endpunkt der Prothese (6) beschränkt;
b) Bestimmung der Mittelebene, welche die Trennung des Brustbeins von den Rippen auf Basis der experimentell bestimmtem Grenzen auf der linken und der rechten Seite der entsprechenden Ebene ermöglicht.

3. Verfahren gemäß einem der vorherigen Ansprüche, worin die grafische präoperative Simulation des erwarteten kosmetischen Resultats der Thoraxwand nach dem Einsetzen der chirurgischen Prothese (6) am Patienten aus den Informationen am Punkt des Einsetzens der Prothese folgendermaßen erhalten wird:
i) dem Punkt der größten Durchbiegung des Brustbeins;
ii) dem Korrekturfaktor des Brustbeins auf Basis des vordersten Punkts der im Grenzbereich vorhandenen Rippen;
iii) und dem stabilisierenden Faktor des Brustbeins, welcher der Verlagerung des Brustbeins nach der Entfernung der Prothese entspricht.

## Revendications

1. Procédé de modelage et de flexion pré-chirurgical, automatique et personnalisé d'une prothèse chirurgicale (6) pour utilisation dans la correction du pectus excavatum, comprenant les étapes suivantes :
a) la segmentation à partir d'images médicales 3D d'un patient, des structures comprenant les côtes et le sternum sur base de références anatomo-chirurgicales ;
b) la détection automatique du point de plus grand enfoncement du sternum sur base des coordonnées 3D des points du sternum ;
c) la définition du cadre limite où se situe l'espace intercostal par :
la définition d'un plan qui inclut le point de plus grand enfoncement du sternum et qui est perpendiculaire au sternum ; la création d'un cuboïde avec l'une des bases butant sur le plan défini et ayant une hauteur égale à la largeur de la prothèse (6); la définition de l'espace intercostal à travers les points des côtes situés à l'intérieur du cuboïde ;
d) correction automatique de l'élévation du sternum à une position prédite pour la correction du pectus excavatum, donnée par le facteur de correction du sternum obtenu à partir du point le plus antérieur des côtes appartenant à l'espace intercostal, cette position permettant une modification manuelle ultérieure par l'utilisateur ;
e) modélisation virtuelle et personnalisée de la prothèse chirurgicale (6) et sélection de la taille standard la plus appropriée de la prothèse à appliquer chez le patient, sur base de la position définie pour le sternum ;
f) le positionnement et la visualisation de la prothèse chirurgicale virtuelle modélisée à partir du point de plus grand enfoncement et de la position finale du sternum ;
g) simulation graphique du résultat cosmétique préopératoire attendu de la paroi thoracique après la mise en place de la prothèse chirurgicale (6) chez le patient ;
h) flexion de la prothèse chirurgicale (6), selon le modèle virtuel, avec surveillance en temps réel et contrôle du comportement mécanique du matériau de la prothèse chirurgicale (6).

2. Procédé selon la revendication précédente, dans lequel la segmentation à partir des images 3D de la structure de côte et de la structure de sternum comprend les étapes suivantes :
a) l'identification du plan coronal, incluant la ligne médio-axillaire, qui limite la position antérieure des côtes et, par conséquent, les points initial et final de la prothèse (6) ;
b) l'identification du plan médial qui, sur base des limites déterminées expérimentalement à gauche et à droite du plan respectif, permet la séparation du sternum des côtes.

3. Procédé selon l'une quelconque des revendications précédentes, dans lequel la simulation graphique pré-chirurgicale du résultat cosmétique attendu de la paroi thoracique, après la mise en place de la prothèse chirurgicale (6) chez le patient, est obtenue à partir d'informations sur le point de placement de la barre obtenu à partir :
i) du point de plus grand enfoncement du sternum ;
ii) du facteur de correction du sternum donné par le point le plus antérieur des côtes présent dans le cadre limite ;
iii) et par le facteur de stabilisation du sternum qui correspond au déplacement du sternum après le retrait de la barre.
